Europäisches Patentamt

European Patent Office

Office européen des brevets

(19)

(11) **EP 0 824 240 B1**

(12) **EUROPÄISCHE PATENTSCHRIFT**

(45) Veröffentlichungstag und Bekanntmachung des Hinweises auf die Patenterteilung:
**04.06.2003 Patentblatt 2003/23**

(51) Int Cl.7: **G06F 19/00**

(21) Anmeldenummer: **97250229.8**

(22) Anmeldetag: **06.08.1997**

(54) **Verfahren und Anordnung zur Bestimmung individualspezifischer Insulinwirkäquivalente körperlicher Belastung**

Method and device for determining individually specific physical strain equivalent to insulin effects

Méthode et dispositif pour la détermination des charges physiques individuelles spécifiques équivalentes à l'effet de l'insuline

(84) Benannte Vertragsstaaten:
**AT CH DE GB LI**

(30) Priorität: **10.08.1996 DE 19632371**

(43) Veröffentlichungstag der Anmeldung:
**18.02.1998 Patentblatt 1998/08**

(73) Patentinhaber:
• **Salzsieder, Eckhard, Dr.**
  **17495 Karlsburg (DE)**
• **Rutscher, Alexander, Dipl.-Ing.**
  **17495 Züssow (DE)**

(72) Erfinder:
• **Salzsieder, Eckhard, Dr.**
  **17495 Karlsburg (DE)**
• **Rutscher, Alexander, Dipl.-Ing.**
  **17495 Züssow (DE)**

(74) Vertreter: **Meyerhöfer, Dietmar, Dipl.-Ing.**
**Patentanwalt,**
**Rudolf-Petershagen-Allee 12**
**17489 Greifswald (DE)**

(56) Entgegenhaltungen:
EP-A- 0 650 695          DD-A- 230 730
DD-A- 277 819            GB-A- 2 218 831
US-A- 4 731 726

• HUTTEN H: "A multicompartment model for open-loop control of glucose in insulin-dependent diabetics" XIIITH INTERNATIONAL KARLSBURG SYMPOSIUM ON PROBLEMS OF DIABETES, DRESDEN, EAST GERMANY, 16-18 OCT. 1989, Bd. 32, Nr. 3-4, ISSN 0169-2607, COMPUTER METHODS AND PROGRAMS IN BIOMEDICINE, JULY-AUG. 1990, NETHERLANDS, Seiten 189-193, XP002052478
• FRANK DEICKERT: "Sport und Diabetes", 1991, SPRINGER VERLAG, BERLIN
• BROWN A.C.: 'Energy Metabolism' PHYSIOLOGY AND BIOPHYSICS Bd. 3, Nr. 20TH, 1973, PHILADELPHIA, Seiten 85 - 104

## EP 0 824 240 B1

**Beschreibung**

Die Erfindung betrifft ein Verfahren und eine Anordnung zur Bestimmung individualspezifischer Insulinwirkäquivalente körperlicher Belastung, die für rechnergestützte Lern- und Trainingsprogramme sowie in der Fort- und Weiterbildung einsetzbar sind. Die Erfindung findet auf dem Gebiet der mikrorechnergestützten Medizintechnik Anwendung, vorzugsweise mittelbar mit der Ergometrie gekoppelt.

Es ist schon frühzeitig bekannt geworden, daß körperliche Belastung aufgrund der blutzuckersenkenden Wirkung eine bedeutsame Rolle in der Stoffwechselkontrolle von Patienten mit Diabetes mellitus zukommt.

Durch Kemmer, F. W.: Prevention of hypoglycermia during exercise in type 1 diabetes, Diabetes Care 15, 1992, S. 1732- 1732, ist deshalb vorgeschlagen worden, bei geplanter körperlicher Belastung die zu erwartenden Effekte vorher auf geeignete Weise abzuschätzen. Hierzu sind verschiedene Verfahren durch Deickert, F.: Sport und Diabetes, Springer Verlag, vorgeschlagen worden, darunter die Empfehlungen der European IDDM Policy Group: Consens guidlines for the management of insulin-dependent (type I) diabetes, Medicom Europe BV, IDF, Brussels, 1993, und die der Amerikanischen Diabetesgesellschaft, Physician's guide to insulin-dependent (type I) diabetes: Diagnosis and Treatment, Alexandria, V.A., Am. Diabetes Assoc., 1988.

Bislang ist das Problem der vorherigen Abschätzung der zu erwartenden Effekte einer geplanten körperlichen Belastung nur durch langwieriges Ausprobieren zu lösen, wobei das prinzipielle Ziel dieses Vorgehens in der Regel darin besteht, eine quantitative Beziehung zwischen körperlicher Belastung und Insulinwirkung aufzufinden, was bedeutet, ein individualspezifisches Insulinwirkäquivalent der körperlichen Belastung zu definieren.

Die durch Schmülling, R.-M. et al.: Exercise and insulin requirements, Horm. Metab. Res. 24, 1990, S. 83-87, und Menzel, R.: Insulin zum Leben, Verlag Gesundheit 1992, S. 78 - 84, bekannt gewordenen Verfahren zur Ermittlung eines auf die körperliche Belastung bezogenen Insulinwirkäquivalents bestimmen zumeist allgemeine, unspezifische Bewegungseinheiten, denen in Abhängigkeit von Dauer, Intensität und Art der körperlichen Belastung gemäß Erfahrungswerten Insulindosen zugeordnet werden. Die Anpassung an den individualspezifischen Trainingszustand erfolgt dann in der Regel rein empirisch.

Alle bekannten Verfahren beinhalten den gemeinsamen Nachteil, daß sie weder eine quantitative Beziehung zwischen aktuellem Trainingszustand, körperlicher Belastung und individueller Wirkung von Insulin herzustellen gestatten noch daß sie im Einzelfall eine vorherige Abschätzung der zu erwartenden quantitativen und qualitativen Effekte einer bestimmten körperlichen Belastung erlauben und somit für rechnergestützte Lern- und Trainingsprogramme nicht geeignet sind.

Durch Brown, A. C. in: Energy Metabolism, Physiology and Biophysisc, Philadelphia, 1973, Band 3, 20. Auflage, S. 85 -104, ist bekannt, daß alle körperlichen Belastungen in verbrauchter Energie und in einem Maß für den Stoffwechsel ausgedrückt werden können. Die beschriebenen Energiebilanzen stehen jedoch weder zur individuell unterschiedlichen Insulinwirkung bei dem diabetischen Probanden noch zu der quantitativen Bestimmung in Beziehung. Außerdem ist nicht beschrieben worden, wie die körperliche Belastung quantitativ Einfluss auf die individualspezifische Wirkprofile des Insulin nimmt.

Durch Hutten, H. in: A multicompartment model for open-loop control of glucose in insulin-dependent diabetes, Computer Methods and Programms in Biomedicine, 32 (1990) S. 189- 193 ist allgemein beschrieben worden, daß bei körperlichen Übungen der Blutglukoseverbrauch gesteigert wird und ein Aktivitätsfaktor für die Berechnung der Insulinsenkung während der Übungen verwendet werden kann. Es ist jedoch weder der Aktivitätsfaktor beschrieben noch für einen gegebenen Probanden bestimmt worden. Zudem läßt die Lehre keinerlei Schlußfolgerungen zu einer Beziehung zwischen einer individuellen Insulinwirkung und der Belastung zu.

Durch DD 230730 A3 ist beschrieben, dass mittels eines Mikrorechners und eines Simulationsprogramms individuelle Stoffwechselparameter durch Approximation des gemessenen Glukosekonzentrationsverlaufes, vorzugsweise mittels nichtlinearer Regressionsanalyse ermittelt und danach die individualspezifischen Glukoseregulationsparameter unter verschiedenen Glukose- und Insulinbelastungsbedingungen des diabetischen Probanden mit einem integrierten mathematischen Algorithmus eines physiologischen Glukoseregulationssystem in Form eines Glukose/Insulin-Stoffwechsel-Modells berechnet werden kann. Körperliche Belastung findet hierbei keine Berücksichtigung.

Durch EP 0650695 ist ein Fahrradergometer mit einer integrierten Mikrorechneranordnung zur Messung, Auswertung und Mittelwertbildung der Belastungsherzfrequenzdaten unter dem Gesichtspunkt eines Konditions- und Fitneßtrainings bekannt geworden. Eine Beziehung der Belastungsherzfrequenz zu einer individuellen Insulinwirkung ist nicht beschrieben worden.

Die Aufgabe der Erfindung besteht daher darin, ein Verfahren und eine Mikrorechneranordnung zur Durchführung des Verfahrens bereit zu stellen, welche gestatten, unabhängig von speziellen mess- und rechentechnischen Voraussetzungen ein individualspezifisches Insulinwirkäquivalent körperlicher Belastung analog den Wirkprofilen von Insulin zu ermitteln mit dem Ziel, ein geeignetes Instrumentarium in die Hand zu bekommen, um Sport und andere körperliche Belastungen hinsichtlich der prospektiven Abschätzung ihrer quantitativen und qualitativen Effekte in rechnergestützte Lern- und Trainingsprogramme einbeziehen zu können.

Erfindungsgemäß wird die Aufgabe dadurch gelöst, daß in zwei Verfahrensschritten die individualspezifischen Insulinwirkäquivalente körperlicher Belastungen, bestehend aus einer Maßzahl und einem dieser zugeordneten Wirkprofil, welche zusammengenommen das Ausmaß, die Dauer und den zeitlichen Verlauf des Effektes in Analogie zur Wirkkinetik von Insulin beschreiben, allein aus der individuellen Belastungsherzfrequenz auf der Grundlage des in DD 277819 A3 bereits beschriebenen Glukose/Insulin-Stoffwechsel-Modells ermittelbar sind.

Im ersten Verfahrensschritt wird das individualspezifische Insulinwirkäquivalent (IWÄ$_{indv}$) für eine definierte körperliche Belastung, bestehend aus der individuellen quantitativen Maßzahl (MA$_{indv}$) und dem dieser Maßzahl zugeordneten Wirkprofil (IWÄ(t)$_{indv}$),welches den zeitlichen Verlauf der insulinähnlichen Effekte dieser Belastung mittels Wirkdauer (WD) und Wirkungsstärke (WS) in Abhängigkeit vom individuellen Trainingszustand beschreibt, bestimmt.

Als eine der Eingangsgrößen dieses Verfahrensschrittes dienen deshalb die Belastungsherzfrequenzen (HF$_m$), die in bekannter Weise mittels definierter Ergometerbelastung ermittelt und vorzugsweise nach drahtloser Übertragung als Eingangsdaten zur Abspeicherung in einer Mikrorechneranordnung zur Verfügung stehen.

Verfahrensgemäß wird mittels dieser Mikrorechneranordnung aus den Eingangsdaten Belastungsherzfrequenz (HF$_m$), die über einem Zeitraum von 30 min nach Beginn bis zum Ende der Ergometerbelastung vorzugsweise im Minutentakt vorliegen, durch Mittelwertbildung die individualspezifische Belastungsherzfrequenz (HF$_{indv}$) berechnet und abgespeichert. Anschließend wird regressiv unter Verwendung dieser individualspezifischen Belastungsherzfrequenz (HF$_{indv}$) die individuelle quantitative Maßzahl (MA$_{indv}$), ausgedrückt als Vielfaches der Wirkung einer internationalen Insulineinheit IE, des individualspezifische Insulinwirkäquivalents (IWÄ$_{indv}$) gemäß der Regressionsgleichung

$$MA_{indv} = K_0 - K_1 * HF_{indv} \tag{1}$$

bestimmt, wobei die beiden Regressionskoeffizienten $K_0$ und $K_1$ den erfindungsgemäßen Zusammenhang zwischen den modellgestützt erzeugten Insulinwirkäquivalenten körperlicher Aktivität (IWÄ) und der Belastungsherzfrequenz (HF) beschreiben. Dabei ist der Regressionskoeffizient $K_0$ der numerische Wert der Schnittstelle der Regressionsgeraden mit der IWÄ-Achse und $K_1$ der numerische Wert des Anstieges der Regressionsgeraden. Die numerischen Werte dieser beiden Regressionskoeffizienten resultieren aus den unabhängig vom erfindungsgemäßen Verfahren bei einer repräsentativen Gruppe von insulinbehandelten Diabetikern unter einer definierten klinischen Testbedingung erhobenen Befunde und sind bei Beginn des erfindungsgemäßen Verfahrens in der Mikrorechneranordnung gespeichert worden.

Das dieser individuellen quantitativen Maßzahl (MA$_{indv}$) zugeordnete Wirkprofil (IWÄ(t)$_{indv}$) der definierten körperlichen Belastung wird dann aus dem durch das Glukose/Insulin-Stoffwechsel-Modell definierten Zusammenhang zwischen der quantitativen Maßzahl (MA$_{indv}$) und der Fläche unter dem zeitabhängigen Verlauf der insulinäquivalenten Wirkung körperlicher Belastung (IWÄ(t)) gemäß der Beziehung

$$MA_{indv} = F_1 * \int_{t_1}^{t_2} IWÄ(t)\, dt \tag{2}$$

mit dem Proportionalitätsfaktor $F_1$ zur Beschreibung der Dosis/Wirkungs-Kennlinie für die Ermittlung des Vielfachen der insulinäquivalenten Wirkung der definierten körperlichen Belastung in Relation zu einer subkutan verabfolgten Insulineinheit bestimmt. Der zeitabhängige Verlauf der insulinäquivalenten Wirkung körperlicher Belastung (IWÄ(t)) wird dabei modellgestützt durch Input/Output-Inversion simuliert mittels iterativer Integration des dem Glukose/Insulin-Stoffwechsel-Modell zugeordneten Differentialgleichungssystems

$$x' = u + CHO(t) \tag{3.1}$$

$$u' = -(b_1 + b_2)u - b_3(y + e) + b_1(b_0 - CHO(t)) \tag{3.2}$$

$$y' = -ky + INS(t) \tag{3.3}$$

$$e' = -k\,e + IW\ddot{A}(t) \tag{3.4}$$

und durch Vergleich des zeitabhängigen Verlaufes der insulinäquivalenten Wirkung körperlicher Belastung (IWÄ(t)) mit dem für eine definierte subkutane Insulinapplikation bekannten Profil der blutzuckersenkenden Wirkung des Insulins INS(t) ermittelt.

Im Differentialgleichungssystem bedeuten x', u', y' und e' Zustandsgrößen, die den Zeitablauf der Blutglukosekonzentration (x), der endogenen Glukosebilanz (u), der Insulinkonzentration im Blut (y) und der insulinäquivalenten Wirkung körperlicher Belastung (e) beschreiben.

Die Größen $b_i$ und k sind Parameter des Differentialgleichungssystems für die endogene Glukoseproduktion ($b_0$), die Größen $b_1$ und $b_2$ sind der Verstärkungsfaktor und die Zeitkonstante des vom Insulin unabhängigen Glukoseumsatzes, die Größe $b_3$ der Insulin-Glukose-Wirkfaktor und die Größe k steht für die Zeitkonstante des Insulinkatabolismus.

Der zeitliche Verlauf der Nahrungsresorption CHO(t), der blutzuckersenkenden Wirkung des subkutan verabfolgten Insulins INS(t) und der insulinäquivalenten Wirkung körperlicher Belastung IWÄ(t) sind die zeitabhängigen Eingangsgrößen des mathematischen Modells des Glukose/Insulin-Stoffwechsels.

Das individualspezifische Insulinwirkäquivalent ($IW\ddot{A}_{indv}$), welches durch die individuelle Maßzahl ($MA_{indv}$) und das dieser Maßzahl zugeordneten Insulinwirkprofil bestimmt wird, ergibt sich aus der Beziehung

$$IW\ddot{A}_{indv} = \int_{t_1}^{t_2} IW\ddot{A}(t)_{indv}\, dt \tag{4},$$

wobei die individuelle Maßzahl ($MA_{indv}$) dem Integral der Fläche unter der Kurve des individuellen zeitabhängigen Wirkungsverlaufs des Insulins, das bedeutet, dem numerischen Wert der Wirkfläche, entspricht.

Sind der individuelle Trainingszustand, der durch die individuelle Belastungsherzfrequenz ($HF_{indv}$) charakterisiert ist, und das individualspezifische Insulinwirkäquivalent ($IW\ddot{A}_{indv}$) bekannt, sind erfindungsgemäß im zweiten Verfahrensschritt die Insulinwirkäquivalente beliebiger körperlicher Belastung ($IW\ddot{A}_{last}$) ermittelbar.

Zu Beginn werden die Werte der Intensität (I) und der Dauer (D) und die Art (A) einer körperlichen Belastung in die Mikrorechneranordnung eingegeben, in dem bereits die individuelle Belastungsherzfrequenz ($HF_{indv}$) abgespeichert ist. Aus den Eingangsgrößen wird gemäß der Beziehung

$$AL_k = A_i * (I * D) \tag{5}$$

zunächst die für die jeweilige körperliche Belastung zu vollbringende Arbeitsleistung ($AL_k$), ausgedrückt in Wh, durch Multiplikation von Intensität (I) und Dauer (D) der körperlichen Belastung berechnet. Die Art der körperlichen Belastung wird in Form des dimensionslosen Multiplikationsfaktors ($A_i$) berücksichtigt, welcher tabellarisch in der erfindungsgemäßen Mikrorechneranordnung abgespeichert ist. Mittels dieses Faktors werden verschiedenen körperlichen Belastungen Faktoren zwischen 0,5 und 5 zugeordnet.

Für die so berechnete Arbeitsleistung ($AL_k$) einer körperlichen Belastung wird dann mittels eines Nomogramms die dieser Arbeitsleistung entsprechende Maßzahl ($MA_{last}$) regressiv gemäß der Gleichung

$$MA_{last} = P_0 + P_1(HF_{indv}) * AL_k \tag{6}$$

ermittelt, wobei die Regressionskoeffizienten $P_0$ und $P_1$ den erfindungsgemäßen Zusammenhang zwischen den modellgestützt erzeugten Insulinwirkäquivalenten körperlicher Belastung (IWÄ) und einer beliebigen körperlichen Arbeitsleistung ($AL_k$) unter Berücksichtigung der individuellen Belastungsherzfrequenz ($HF_{indv}$) beschreiben.

Die numerischen Werte für Regressionskoeffizienten $P_0$ und $P_1$, die in der erfindungsgemäßen Mikrorechneranordnung erzeugt und gespeichert werden, resultieren aus dem Anstieg der Regressionsgeraden der jeweiligen Probanden zwischen dem Punkt $P_0 = 0$ bei $AL_k = 0$ und IWÄ = 0 und dem Punkt $P_1(HF_{indv})$, wobei der Anstieg der Regressionsgeraden der Probenden aus dem zuvor im ersten Verfahrensschritt bei einer definierten Fahrradergometerbelastung von 45 Wh ermittelten individuellen Belastungsherzfrequenz ($HF_{indv}$), und dem dieser Belastungsherzfrequenz zugeordneten individuellen Maßzahl ($MA_{indv}$) ermittelt wird.

Der Einfluss der individuellen Belastungsherzfrequenz ($HF_{indv}$) wird nach Maßgabe der Gleichung (6) dadurch realisiert, daß der durch den Regressionskoeffizienten $P_1$ bestimmte Anstieg der Regressionsgeraden zwischen der körperlichen Arbeitsleistung ($AL_k$) und der dieser Arbeitsleistung entsprechende Maßzahl ($MA_{last}$) funktional von der individuellen Belastungsherzfrequenz ($HF_{indv}$) abhängt und somit individualspezifisch zugeordnet ist.

Das dieser belastungsspezifischen Maßzahl ($MA_{last}$) zugeordnete Wirkprofil ($IWÄ(t)_{last}$) ist dann analog dem Vorgehen im ersten Verfahrensschritt gemäß der Beziehung

$$MA_{last} = F_1 * \int_{t_1}^{t_2} IWÄ(t)dt \qquad\qquad (7)$$

unter Verwendung des dem Glukose/Insulin-Stoffwechsel-Modell zugeordneten Differentialgleichungssystems (3.1 bis 3.4) bestimmbar und aus den beiden Größen schließlich prospektiv das individualspezifische belastungsabhängige Insulinwirkäquivalent

$$IWÄ_{last} = \int_{t_1}^{t_2} IWÄ(t)_{last}dt \qquad\qquad (8)$$

einer beliebigen körperlichen Belastung ermittelbar.

Die erfindungsgemäße Mikrorechneranordnung zur Durchführung des Verfahrens ist vorzugsweise in einem Mikrorechnersystem integriert, welches mit einem Fahrradergometer gekoppelt ist.

Die Mikrorechneranordnung zur Bestimmung des individualspezifischen Insulinwirkäquivalents auf Basis der Belastungsherzfrequenz besteht aus einer ersten Kettenschaltung, die in Serie aus einem ersten Input-Modul, einem Herzfrequenz-Modul, einem Regressions-Modul, einem ersten Differentialgleichungs-Modul und einem ersten Output-Modul gebildet ist und parallel zur ersten Kettenschaltung aus einer zweiten Kettenschaltung zur Bestimmung des individualspezifischen Insulinwirkäquivatents beliebiger körperlicher Aktivitäten, die aus einem zweiten Input-Modul, einem Aufbereitungs-Modul, einem Nomogramm-Modul, einem zweiten Differentialgleichungs-Modul und einem zweiten Output-Modul aufgebaut ist, wobei der Ausgang des Herzfrequenz-Moduls der ersten Kettenschaltung auch an den zweiten Eingang des Nomogramm-Moduls der zweiten Kettenschaltung geführt ist.

Zu beiden Kettenschaltungen ist ein Modellreferenz-Modul zugeordnet, dessen erster Ausgang in der ersten Kettenschaltung an den zweiten Eingang des Regressions-Moduls und dessen zweiter Ausgang an den zweiten Eingang des ersten Differentialgleichungs-Moduls geführt ist. Der dritte Ausgang des Modellreferenz-Moduls ist in der zweiten Kettenschaltung an den zweiten Eingang des zweiten Differentialgleichungs-Moduls und der vierte Ausgang des Modellreferenz-Moduls an den dritten Eingang des Nomogramm-Moduls geschaltet.

An den zwei Eingängen eines ersten Input-Moduls der Anordnung werden die Eingangsdaten Intensität ($I_D$) und Dauer ($D_D$) der definierten Ergometerbelastung eingegeben. Von dem Herzfrequenz-Modul, das dem ersten Input-Modul nachgeschaltet ist, werden die Eingangsdaten in Form der während einer definierten Ergometerbelastung aufeinander folgend abgespeicherten Herzfrequenzwerte ($HF_m$) übernommen und zwischengespeichert. Anschließend wird erfindungsgemäß in diesem Herzfrequenz-Modul aus den Einzelwerten durch Mittelwertbildung die individualspezifische Belastungsfrequenz ($HF_{indv}$) ermittelt und als Signal an den ersten Eingang des Regressions-Moduls und an den zweiten Eingang des Nomogramm-Moduls geführt. Der erste Ausgang des Modellreferenz-Moduls stellt die für die Regressionsrechnung erforderlichen Werte des Insulinwirkäquivalents körperlicher Belastung (IWÄ) an den zweiten Eingang des Regressions-Moduls bereit. Der Ausgang des Regressions-Moduls übergibt an den ersten Eingang des ersten Differenzgleichungs-Moduls die individuelle Maßzahl ($MA_{indv}$). Über seinen zweiten Eingang ist das erste Differentialgleichungs-Modul zur Übergabe der im Modellreferenz-Modul abgespeicherten Referenzwerte der blutzuckersenkenden Wirkung des subkutan verabfolgten Insulins (INS(t)) und der numerischen Modellparameter $b_1$ und k mit dem zweiten Ausgang des Modellreferenz-Moduls verbunden. Der Ausgang des ersten Differentialgleichungs-Moduls geht auf den Eingang des ersten Output-Moduls, in welchem die abschließende Ermittlung, Speicherung und die Ausgabe des resultierenden individualspezifischen Insulinwirkäquivalents ($IWÄ_{indv}$) der definierten Ergometerbelastung erfolgt.

Über das Input-Modul der zweiten Kettenschaltung, die der Bestimmung des individualspezifischen Insulinwirkäquivalents beliebiger körperlicher Aktivitäten dient, werden die Daten Intensität (I), Dauer (D) und Art ($A_i$) einer beliebigen körperlichen Belastung eingegeben und an das nachgeschaltete Aufbereitungs-Modul weitergeleitet. Die im Aufbereitungs-Modul aus den Daten Intensität (I), Dauer (D) und Art ($A_i$) der körperlichen Belastung ermittelte Arbeitsleistung ($A_k$) wird an den ersten Eingang des Monogramm-Moduls übergeben. Die für die Berechnung der belastungsspezifischen Maßzahl ($MA_{last}$) erforderlichen Insulinwirkäquivalente (IWÄ) werden über den dritten Eingang des Nomogramm-Moduls bereitgestellt, welcher hierzu mit dem dritten Ausgang des Modellreferenz-Moduls verbunden ist. Die Übergabe der belastungsspezifischen Maßzahl ($MA_{last}$) als Signal vom Nomogramm-Modul an das zweite Differentialgleichungs-Modul erfolgt über dessen ersten Eingang. Der zweite Eingang des zweiten Differentialgleichungs-Moduls ist mit dem vierten Ausgang des Modellreferenz-Moduls zur Bereitstellung der für die weitere Berechnung erforderlichen Referenzwerte der blutzuckersenkenden Wirkung subkutan verabfolgten Insulins (INS(t)) und der Modellparameterwerte $b_1$ und $k_1$ verbunden. Nach der im zweiten Differentialgleichungs-Modul durchgeführten Bestimmung des belastungsspezifischen Insulinwirkäquivalents ($IWÄ(t)_{last}$) werden diese an das zweite Output-Modul zur abschließenden Ermittlung, Speicherung und Ausgabe der resultierenden individualspezifischen Insulinwirkäquivalente beliebiger körperlicher Belastung ($IWÄ)_{last}$) übergeben.

Die Erfindung soll nachstehend anhand eines Ausführungsbeispiels näher erläutert werden. In der dazugehörigen Zeichnung sind dargestellt:

Fig. 1 schematische Darstellung der Beziehung zwischen der individuellen Belastungsherzfrequenz und der dieser erfindungsgemäß zugeordneten Insulinwirkäquivalente

Fig. 2 erfindungsgemäße Mikrorechneranordnung

Fig. 3 Darstellung der Insulinwirkäquivalente für die Probanden Pb1, Pb2

Fig. 4 Darstellung der Wirkprofile, die aus den Maßzahlen 0,9 und 5,1 nach dem bekannten Verfahren gemäß DD 277819 A3 bestimmt sind

Fig. 5 Darstellung der Beziehung zwischen Arbeitsleistung und IWÄ für die Probanden Pb1, Pb2

Fig. 6 Darstellung der Beziehung zwischen Wirkstärke WS und Wirkdauer WD zu den IWÄ für die Probanden Pb1, Pb2

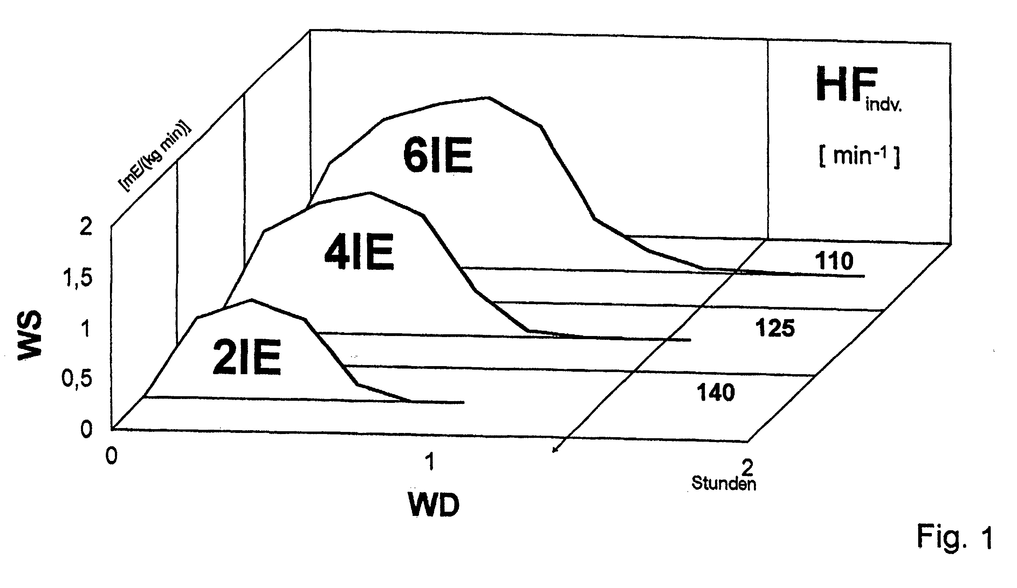

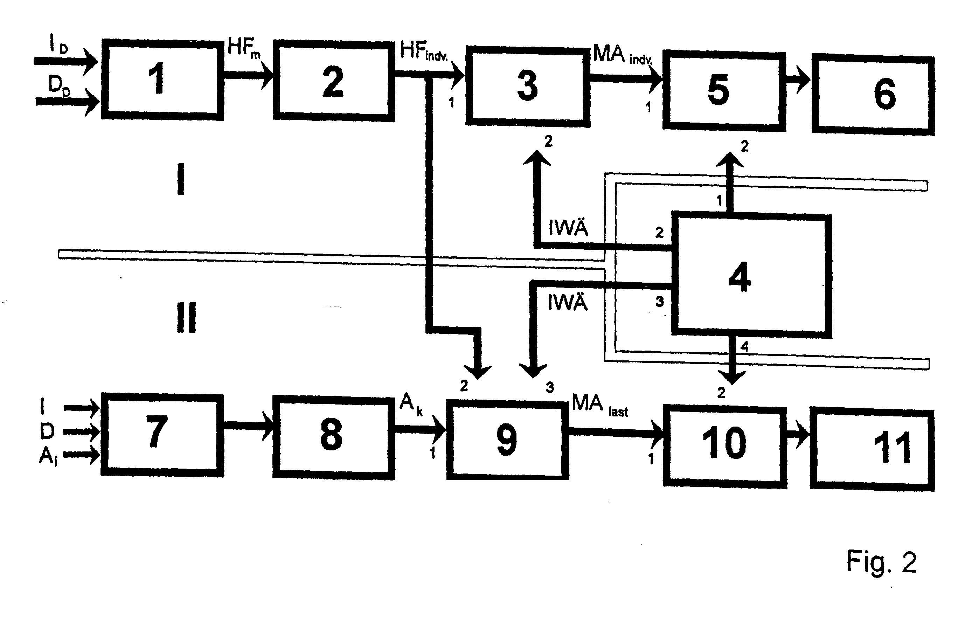

Für zwei Modellfälle Proband Pb1 und Pb2 werden die individualspezifischen Insulinwirkäquivalente ($IWÄ_{indv}$) gemäß Fig. 1 sowie die Insulinwirkäquivalente für einen Waldlauf ($IWÄ_{last}$) von ca. 40 min Dauer über eine Distanz von etwa 12 km ermittelt. Die mittels handelsüblichen Fahrrad-Ergometers mit angeschlossenem Mikrorechnersystem und unter einer Belastung von einer Dauer $D_D = 30$ min und einer Intensität $I_D = 90$ Watt abgespeicherten Belastungsfrequenzdaten $HF_m$ werden als Eingangsdaten in die erfindungsgemäßen Mikrorechneranordnung gemäß Fig. 2 eingegeben.

Diese Mikrorechneranordnung kann in das Mikrorechnersystem des Fahrradergometers integriert sein und besteht aus dem Input-Modul 1, dem Herzfrequenz-Modul 2, dem Regressions-Modul 3, dem ersten Differentialgleichungs-Modul 5 und dem ersten Output-Modul 6 in der ersten Kettenschaltung und parallel dazu die zweiten Kettenschaltung mit dem Input-Modul 7, dem Aufbereitungs-Modul 8, dem Nomogramm-Modul 9, dem zweiten Differentialgleichungs-Modul 10 und dem zweiten Output-Modul 11. Beide Kettenschaltungen sind mit den Ausgängen des Modellreferenz-Moduls 4 verkoppelt.

Aus den abgespeicherten Eingangsdaten $HF_m$ werden mittels dieser erfindungsgemäßen Anordnung im ersten Verfahrensschritt für den Modellfall Proband Pb1 eine individuelle Belastungsherzfrequenz $HF1_{indv}$ von 147 Pulsschlägen / min und für den Modellfall Proband Pb2 eine individuelle Belastungsherzfrequenz $HF2_{indv}$ von 118 Pulsschlägen /min ermittelt.

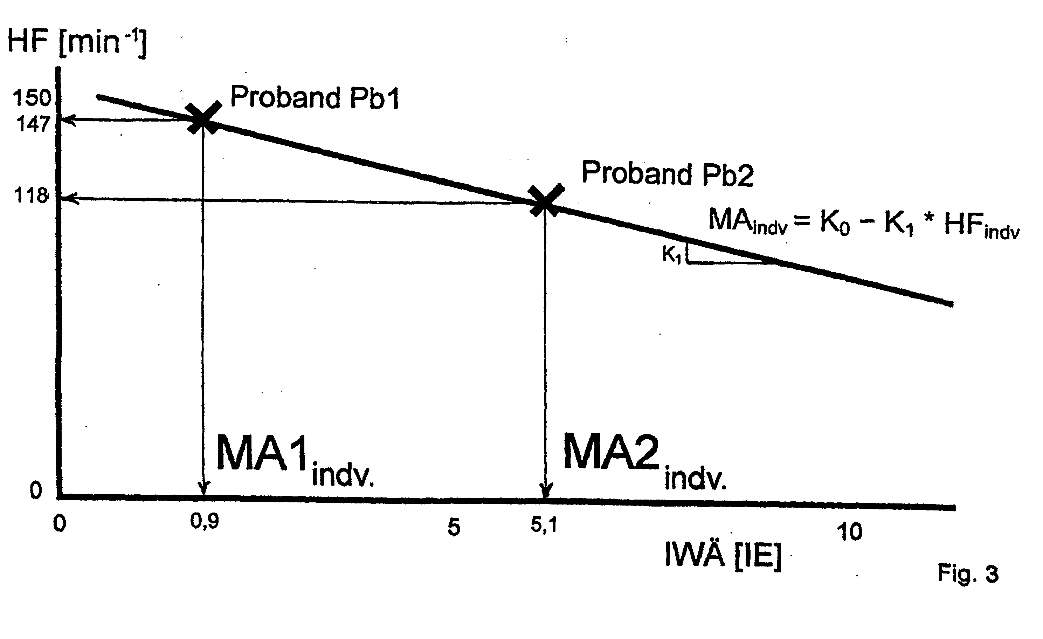

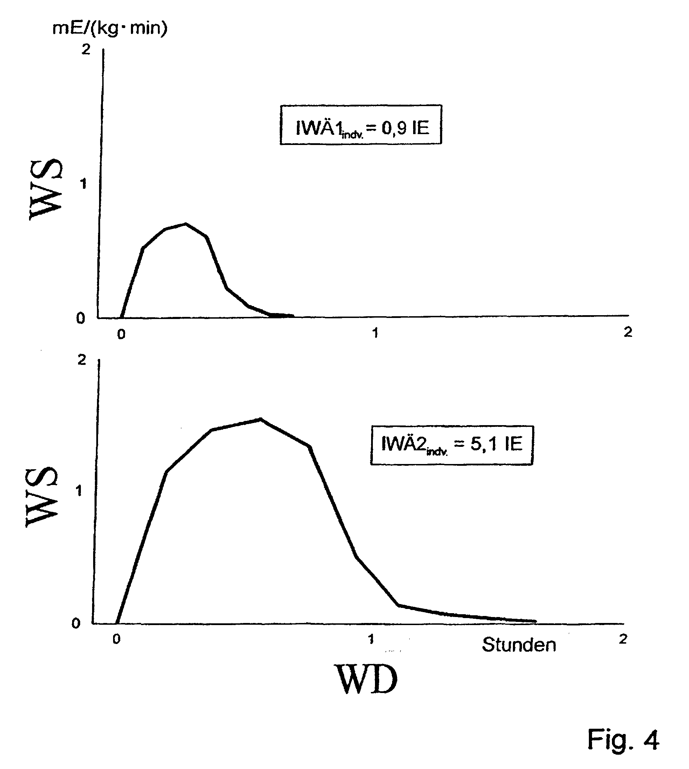

Diesen individuelle Belastungsherzfrequenzen $HF1_{indv}$ für Proband Pb1 bzw. $HF2_{indv}$ für Proband Pb2 sind mittels Regressionsgleichung (1) in Form der Regressionsgeraden $MA_{indv} = k_0 - K_1 * HF_{indv}$ die quantitative Maßzahlen der individualspezifischen Insulinwirkäquivalente $MA1_{indv} = 0,9$ IE für Proband Pb1 bzw. $MA2_{indv} = 5,1$ IE für Proband Pb2 gemäß Fig. 3 zugeordnet, wobei $K_0$ in Fig. 3 grafisch nicht dargestellt ist, da der Schnittpunkt der Regressionsgeraden mit der IWÄ-Achse im bildlich nicht dargestellten Bereich liegt. Aus Gleichung (2) ergeben sich modellgestützt durch Input/Output-Inversion mittels iterativen Integration des dem Glukose/Insulin-Stoffwechsel-Modell zugeordneten Differentialgleichungssystems nach Gleichungen (3.1) bis (3.4) sowie anschließender Anwendung von Gleichung (4) die in Fig. 4 dargestellten individualspezifischen Insulinwirkäquivalente $IWÄ1_{indv}$ für Proband Pb1 bzw. $IWÄ2_{indv}$ für Proband Pb2 für die definierte Belastung IWÄ(t).

Unter Verwendung der so ermittelten individuelle Belastungsherzfrequenzen $HF1_{indv}$ für Proband Pb1 bzw. $HF2_{indv}$ für Proband Pb2 und der diesen zugeordneten individualspezifischen Insulinwirkäquivalente $IWÄ1_{indv}$ für Proband Pb1 bzw. $IWÄ2_{indv}$ für Proband Pb2 sind nunmehr im zweiten Verfahrensschritt die für den jeweiligen Modellfall zutreffenden Insulinwirkäquivalente $IWÄ1_{last}$ bzw. $IWÄ2_{last}$ für den Waldlauf bestimmbar.

Als Belastungswerte werden die Intensität 1 = 12 km/h, die Dauer D = 40 min und die Art $A_i$ = Laufen in das Input-Modul 7 der erfindungsgemäßen Mikrorechneranordnung eingegeben. Gemäß der Gleichung (5) wird aus diesen Daten mittels des Aufbereitungs-Moduls 8 die zu vollbringende Arbeitsleistung zu $AL_k = 82$ Wh berechnet.

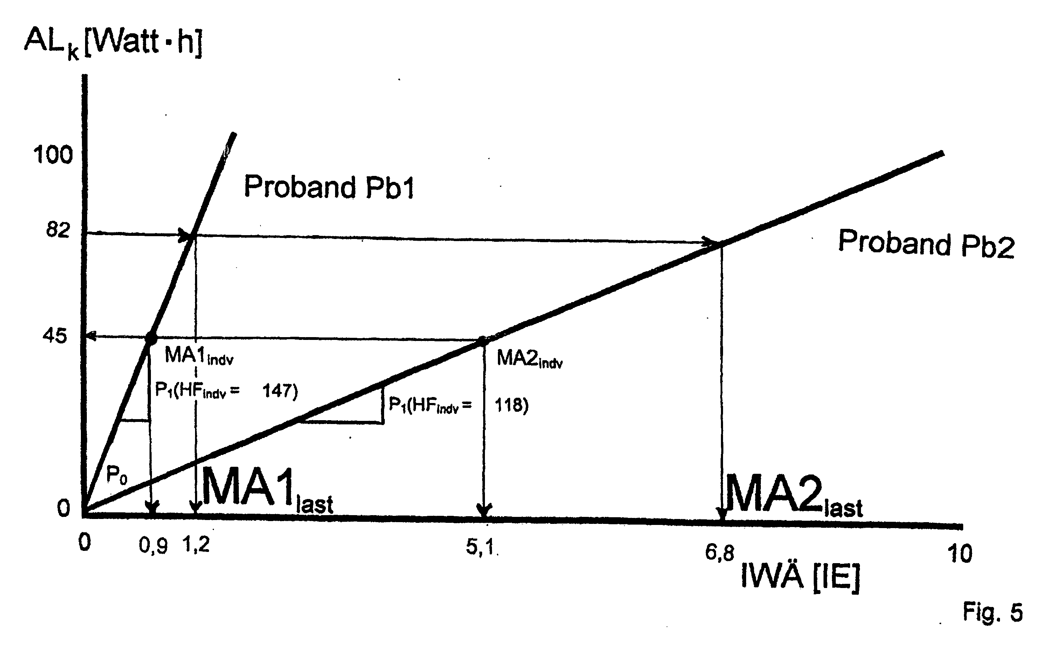

Es werden gemäß Fig. 5 mit Hilfe der Gleichung (6) in Form der Regressionsgeraden $MA_{last} = P_0 + P_1(HF_{indv})$

* $AL_K$ für die beliebige Belastung der Modellfälle Proband Pb1 und Pb2 im Nomogramm-Modul 9 der Mikrorechneranordnung die individuelle Maßzahl $MA_{last}$ bestimmt.

Für den Probanden Pb1 resultieren die numerischen Werte für die Regressionskoeffizienten $P_0$ und $P_1$, die im Regressionsmodul 3 erzeugt und danach gespeichert werden, aus dem Anstieg der Regressionsgeraden zwischen dem Punkt $P_0$ bei $AL_K = 0$, $IWÄ = 0$ und dem Punkt $P_1(HF_{indv} = 147)$.

Der Anstieg der Regressionsgeraden ist zuvor bereits im ersten Verfahrensschritt während der definierten Fahrrad-Ergometerbelastung $AL_K = 45$ Wh durch die ermittelte individuellen Belastungsfrequenz $HF_{indv} = 147$ Pulsscahläge/min und dem dieser Belastungsfrequenz zugeordneten individuellen Maßzahl $MA1_{indv} = 0,9$ IE errechnet worden. Bei einer körperlichen Arbeitsleistung $AL_K = 82$ Wh des Probanden Pb1 ist mit dem Anstieg der Regressionsgeraden die individuelle Maßzahl $MA1_{Last} = 1,2$ IE festgestellt.

Analog resultieren für den Modellfall Proband Pb2 die numerischen Werte für die Regressionskoeffizienten $P_0$ und $P_1$ aus dem Anstieg der Regressionsgeraden zwischen dem Punkt $P_0$ bei $AL_K = 0$, $IWÄ = 0$ und dem Punkt $P_1$ $(HF_{indv} = 118)$.

Der Anstieg der Regressionsgeraden ist ebenfalls zuvor im ersten Verfahrensschritt während der definierten Fahrradergometerbelastung $AL_K = 45$ Wh durch die ermittelte individuellen Belastungsfrequenz $HF_{indv} = 118$ Pulsschläge/min und dem dieser Belastungsfrequenz zugeordneten individuellen Maßzahl $MA2_{indv} = 5,1$ IE errechnet worden. Bei einer körperlichen Arbeitsleistung $AL_K = 82$ Wh des Probanden Pb2 ist mit dem Anstieg der Regressionsgeraden die individuelle Maßzahl $MA1_{Last} = 6,8$ IE festgestellt.

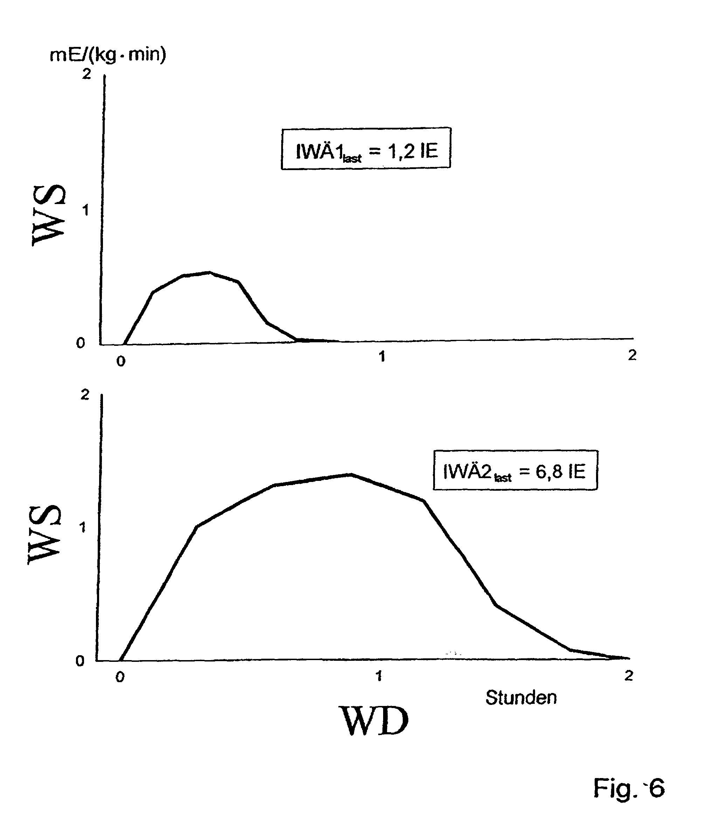

Unter Anwendung der Input/Output-Inversion mittels iterativen Integration des dem Glukose/Insulin-Stoffwechsel-Modell zugeordneten Differentialgleichungssystems nach Gleichungen (3.1) bis (3.4) und den Gleichungen (7) und (8) werden dann die belastungsabhängigen individualspezifischen Insulinwirkäquivalente $IWÄ1_{last}$ bzw. $IWÄ2_{last}$ gemäß Fig. 6 bei den Modellfällen Proband Pb1 und Pb2 ermittelt, die nunmehr zur prospektiven individualspezifischen Abschätzung der zu erwartenden Effekte unter unterschiedlichen Bedingungen vorzugsweise in ein rechnergestütztes Lern- und Trainingsprogramm eingeben werden können.

**Patentansprüche**

1. Verfahren zur Bestimmung individualspezifischer Insulinwirkäquivalente körperlicher Belastung, **dadurch gekennzeichnet, dass** zu den Werten der Intensität, Dauer und Art einer körperlichen Belastung in einem ersten Verfahrensschritt aus Belastungsherzfrequenzdaten als verfahrensgemäße Eingangsdaten für eine Mikrorechneranordnung mittels Mittelwertbildung eine individualspezifische Belastungsherzfrequenz $(HF_{indv})$ abgeleitet und in der Mikrorechneranordnung abgespeichert wird, dass mittels dieser individualspezifischen Belastungsherzfrequenz rechnergestützt das individualspezifische Insulinwirkäquivalent $(IWÄ_{indv})$ für eine definierte körperliche Belastung, bestehend aus einer quantitativen Maßzahl $(MA_{indv})$ und dem dieser Maßzahl zugeordneten Insulinwirkprofit $(IWÄ(t)_{indv})$, dadurch bestimmt wird, dass unter Verwendung der individualspezifischen Belastungsherzfrequenz die quantitative Maßzahl $(MA_{indv})$, ausgedrückt als Vielfaches der Wirkung einer internationalen Insulineinheit, des individualspezifischen Insulinwirkäquivalents mittels Regressionsgleichung

$$MA_{indv} = K_0 - K_1 * HF_{indv}$$

errechnet wird, wobei der Regressionskoeffizient $K_0$ der numerische Wert der Schnittstelle der Regressionsgeraden mit der IWÄ-Achse und Regressionskoeffizient $K_1$ der numerische Wert des Anstieges der Regressionsgeraden bedeuten, und diese vor Beginn des Verfahrens unter definierter klinischer Testbedingung bei insulinbehandelten Diabetikern ermittelt wurden und die numerischen Werte der Regressionskoeffizienten in der Mikrorechneranordnung gespeichert sind, dass Regressionskoeffizienten $K_0$ und $K_1$ den Zusammenhang zwischen den mittels Glukose/Insulin-Stoffwechsel-Models gestützt erzeugten Insulinwirkäquivalenten (IWÄ) bei einer definierten körperlichen Belastung und der Belastungsherzfrequenz beschreiben, und dass das dieser individuellen quantitativen Maßzahl zugeordneten Wirkprofil $(IWÄ(t)_{indv})$ des Ausmaßes, der Dauer und des zeitlichen Verlaufes der insulinähnlichen Effekte aus dem durch das Glukose/Insulin-Stoffwechsel-Modell definierten Zusammenhang zwischen der quantitativen Maßzahl und der Fläche unter dem zeitabhängigen insulinäquivalenten Wirkungsverlauf der körperlichen Belastung (IWÄ(t)) gemäß der Beziehung

$$MA_{indv} = F_1 * \int_{t_1}^{t_2} IW\ddot{A}(t)\, dt$$

mit dem Proportionalitätsfaktor $F_1$ zur Beschreibung der Dosis/Wirkungs-Kennlinie für die Ermittlung des Vielfachen der insulinäquivalenten Wirkung der definierten körperlichen Belastung in Relation zu einer verabfolgten Insulineinheit bestimmt werden, dass der zeitabhängige insulinäquivalente Wirkungsverlauf der körperlichen Belastung (IWÄ(t)) modellgestützt durch Input/Output-Inversion mittels iterativer Integration des dem Glukose/Insulin-Stoffwechsel-Modell zugeordneten Differentialgleichungssystems

$$x' = u + CHO(t),$$

$$u' = - (b_1 + b_2)u - b_3\,(y + e) + b_1(b_0 - CHO(t)),$$

$$y' = - k\,y + INS(t),$$

$$e' = - k\,e + IW\ddot{A}(t),$$

wobei als Zustandsgrößen $x$ = Blutglukosekonzentrationsverlauf, $u$ = endogene Glukosebilanz, $y$ = Insulinkonzentration im Blut und $e$ = die insulinäquivalente Wirkung körperlicher Belastung sowie als die zeitabhängigen Eingangsgrößen des Modells
CHO(t) = zeitliche Verlauf der Nahrungsresorption,
INS(t) = zeitlicher Verlauf der blutzuckersenkenden Wirkung des Insulins,
$b_i$ und $k$ = Parameter für die endogene Glukoseproduktion $(b_0)$,
$b_1$ und $b_2$ = Verstärkungsfaktor und Zeitkonstante des insulinabhängigen Glukoseumsatzes,
$b_3$ = insulinstimulierende Glukoseverwertung und
$k$ = Zeitkonstante des Insulinkatabolismus
definiert sind, simuliert und durch Vergleich des zeitabhängige insulinäquivalente Wirkungsverlaufes der körperlichen Belastung (IWÄ(t)) mit dem für eine definierte subkutane Insulinapplikation bekannten Profil der blutzuckersenkenden Wirkung des Insulins INS(t) ermittelt wird,
und dass das individualspezifische Insulinwirkäquivalent (IWÄ$_{indv}$) für die definierte körperliche Belastung aus der Beziehung

$$IW\ddot{A}_{indv} = \int_{t_1}^{t_2} IW\ddot{A}(t)_{indv}\, dt$$

berechnet wird, wobei die individuelle quantitative Maßzahl dem Integral der Fläche unter der Kurve des individuellen zeitabhängigen Wirkungsverlaufs des Insulin entspricht,
dass in einem zweiten Verfahrensschritt das Insulinwirkäquivalent einer beliebigen körperlichen Belastung (IWÄ$_{last}$), bestehend aus einer quantitativen Maßzahl (MA$_{last}$) und dem dieser Maßzahl zugeordneten Insulinwirkprofil (IWÄ(t)$_{last}$), dadurch ermittelt wird, dass zu Beginn die Werte der Intensität (I) und der Dauer (D) und die Art (A) einer körperlichen Belastung in die Mikrorechneranordnung eingegeben wird, dass aus den Eingangsgrößen gemäß der Beziehung

$$AL_k = A_i * (I * D)$$

zunächst die für die jeweilige körperliche Aktivität zu vollbringende Arbeitsleistung (AL$_k$), ausgedrückt in Wattstunden, durch Multiplikation von Intensität (I) und Dauer (D) der körperlichen Belastung berechnet wird, wobei die Art der körperlichen Belastung in Form des dimensionslosen Multiplikationsfaktors (A$_i$) berücksichtigt wird, welcher tabellarisch in der Mikrorechneranordnung abgespeichert ist, dass für die so berechnete Arbeitsleistung (AL$_k$) einer körperlichen Belastung mittels eines Nomogramms die dieser Arbeitsleistung entsprechende Maßzahl (MA$_{last}$) regressiv gemäß der Gleichung

$$MA_{last} = P_0 + P_1(HF_{indv}) * AL_k$$

ermittelt wird, wobei die numerischen Werte für die Regressionskoeffizienten $P_0$ und $P_1$, die in der Mikrorechneranordnung erzeugt und gespeichert werden, aus dem Anstieg der Regressionsgeraden des jeweiligen Probanden zwischen dem Punkt $P_0$ bei AL$_k$ = 0, IWÄ = 0 und dem Punkt $P_1$(HF$_{indv}$) resultieren, wobei der Anstieg der Regressionsgeraden aus dem zuvor im ersten Verfahrensschritt bei einer definierten Fahrradergometerbelastung von 45 Wattstunden ermittelten individuellen Belastungsherzfrequenz und dem dieser Belastungsherzfrequenz zugeordneten individuellen Maßzahl (MA$_{indv}$) ermittelt wird, dass die Regressionskoeffizienten $P_0$ und $P_1$ bei beliebiger körperlicher Belastung den Zusammenhang zwischen den modellgestützt erzeugten Insulinwirkäquivalenten körperlicher Belastung (IWÄ) und einer beliebigen körperlichen Arbeitsleistung (AL$_k$) unter Berücksichtigung der individuellen Belastungsherzfrequenz (HF$_{indv}$) und der Intensität, der Dauer und der Art der jeweiligen Belastung beschreiben, dass der Einfluss der individuellen Belastungsherzfrequenz (HF$_{indv}$) nach Maßgabe der Gleichung für die belastungsspezifische Maßzahl (MA$_{last}$) dadurch realisiert wird, dass der durch den Regressionskoeffizienten $P_1$ bestimmte Anstieg der Regressionsgeraden zwischen der körperlichen Arbeitsleistung (AL$_k$) und der dieser Arbeitleistung entsprechende Maßzahl (MA$_{last}$) funktional von der individuellen Belastungsherzfrequenz (HF$_{indv}$) abhängt und somit individualspezifisch zugeordnet ist und dass dieser belastungsspezifischen Maßzahl (MA$_{last}$) zugeordnete Wirkprofil (IWÄ(t)$_{last}$) analog dem Vorgehen im ersten Verfahrensschritt gemäß der Beziehung

$$MA_{last} = F_1 * \int_{t_1}^{t_2} IW\ddot{A}(t)dt$$

unter Verwendung des dem Glukose/Insulin-Stoffwechsel- Modell zugeordneten Differentialgleichungssystems bestimmt und aus den beiden Größen schließlich prospektiv das individualspezifische belastungsabhängige Insulinwirkäquivalent

$$IW\ddot{A}_{last} = \int_{t_1}^{t_2} IW\ddot{A}(t)_{last}dt$$

einer beliebigen körperlichen Belastung ermittelt wird.

2. Mikrorechneranordnung zur Durchführung des Verfahrens nach Anspruch 1, **dadurch gekennzeichnet, daß** ihre erste Kettenschaltung, die zur Bestimmung des individualspezifischen Insulinwirkäquivalents auf Basis der Belastungsherzfrequenz dient, in Reihe aus einem ersten Input-Modul (1), einem Herzfrequenz-Modul (2), einem Regressions-Modul (3), einem ersten Differentialgleichungs-Modul (5) und einem ersten Output-Modul (6) besteht und parallel zu dieser Kettenschaltung eine zweite Kettenschaltung zur Bestimmung des individualspezifischen Insulinwirkäquivalents beliebiger körperlicher Aktivitäten angeordnet ist, die in Reihe aus einem zweiten Input-Modul (7), einem Aufbereitungs-Modul (8), einem Nomogramm-Modul (9), einem zweiten Differentialgleichungs-Modul (10) und einem zweiten Output-Modul (11) gebildet ist, daß der Ausgang des Herzfrequenz-Moduls (2) an den zweiten Eingang des Nomogramm-Moduls (9) geführt ist, und daß je ein Ausgang eines Modellreferenz-Moduls (4) an einen Eingang des Regressions-Moduls, des ersten und zweiten Differentialgleichungs-Moduls und des Nomogramm-Moduls geschaltet sind.

**Claims**

1. Method for determining individually specific physical activities equivalents to insulin effects, **characterized in that** for the values of the intensity, duration and nature of a physical exercise in a first step of the method an individual-specific exercise heart rate ($HR_{indv}$) is derived from exercise heart rate data as methodological input data for a microcomputer arrangement by means of averaging, and is stored in the microcomputer arrangement, **in that** this individual-specific exercise heart rate is used for computer-assisted determination of the individual-specific insulin effect equivalent ($IEE_{indv}$) for a defined physical exercise, consisting of a quantitative numerical measure ($MA_{indv}$) and of the insulin effect profile ($IEE(t)_{indv}$) assigned to this numerical measure, by using the individual-specific exercise heart rate to calculate the quantitative numerical measure ($MA_{indv}$) expressed as multiple of the effect of an international insulin unit, of the individual-specific insulin effect equivalent by means of the regression equation

$$MA_{indv} = K_0 - k_1 * HR_{indv},$$

where the regression coefficient $K_0$ is the numerical value of the intercept of the regression line with the IEE axis and regression coefficient $K_1$ is the numerical value of the gradient of the regression line, and the latter have been found before beginning the method under defined clinical test condition on insulin-treated diabetics, and the numerical values of the regression coefficients are stored in the microcomputer arrangement, **in that** regression coefficients $K_0$ and $K_1$ describe the connection between the insulin effect equivalents (IEE), generated with the assistance of the glucose/insulin metabolism model, at a defined physical exercise and the exercise heart rate, and **in that** the effect profile ($IEE(t)_{indv}$), assigned to this individual quantitative numerical measure, of the extent, of the duration and of the time course of the insulin-like effects are determined from the connection, defined via the glucose/insulin metabolism model, between the quantitative numerical measure and the area under the time-dependent insulin-equivalent effect course of the physical exercise (IEE(t)) according to the relation

$$MA_{indv} = F_1 * \int_{t_1}^{t_2} IEE(t)\, dt$$

with the proportionality factor F1 to describe the dose/response characteristic to find the multiple of the insulin-equivalent effect of the defined physical exercise in relation to an administered insulin unit, **in that** the time-dependent insulin-equivalent effect course of the physical exercise (IEE(t)) is simulated on the basis of the model by input/output inversion by means of iterative integration of the system of differential equations assigned to the glucose/insulin metabolism model

$$x' = u + CHO(t),$$

$$u' = -(b_1 + b_2)u - b_3 (y + e) + b_1(b_0 - CHO(t)),$$

$$y' = -k\, y + INS(t),$$

$$e' = -k\, e + IEE(t),$$

where the state variables are defined as x = blood glucose concentration course, u = endogenous glucose balance, y = insulin concentration in the blood and e = the insulin-equivalent effect of physical exercise, and the time-dependent input variables of the model are defined as
CHO(t) = time course of the absorption of food,
INS(t) = time course of the blood glucose-lowering effect of insulin,
$b_i$ and k = parameters for endogenous glucose production ($b_0$),
$b_1$ and $b_2$ = gain and time constant of the insulin-dependent glucose turnover,

$b_3$ = insulin-stimulating glucose utilization and
k = time constant of insulin catabolism,
and is found by comparison of the time-dependent insulin-equivalent effect course of the physical exercise (IEE (t)) with the profile, known for a defined subcutaneous administration of insulin, of the blood glucose-lowering effect of insulin INS(t),
and **in that** the individual-specific insulin effect equivalent ($IEE_{indv}$) is calculated for the defined physical exercise from the relation

$$IEE_{indv} = \int_{t_1}^{t_2} IEE(t)_{indv}\, dt$$

where the individual quantitative numerical measure corresponds to the integral of the area under the curve of the individual time-dependent insulin effect course, **in that** in a second step of the method the insulin effect equivalent of any physical exercise ($IEEl_{load}$), consisting of a quantitative numerical measure ($MA_{load}$) and of the insulin effect profile ($IEE(t)_{load}$) assigned to this numerical measure is found by initially inputting the values of the intensity (I) and the duration (D) and the nature (A) of a physical exercise into the microcomputer arrangement, **in that** initially the work ($W_p$) to be completed for the particular physical activity, expressed in watt-hours, is calculated by multiplying the intensity (I) and duration (D) of the physical exercise from the input variables according to the relation Wp = Ai * (1 * D), with the nature of the physical exercise being taken into account in the form of the dimensionless multiplication factor ($A_i$), which is stored in tabular form in the microcomputer arrangement, **in that** the numerical measure ($MA_{load}$) corresponding to this work is found regressively by means of a nomogram in accordance with the equation

$$MA_{load} = P_0 + P_1(HR_{indv}) * W_p$$

for the work ($W_p$), calculated in this way, of a physical exercise, where the numerical values for the regression coefficients $P_0$ and $P_1$, which are generated and stored in the microcomputer arrangement, results from the gradient of the regression line of the particular subject between the point $P_0$ and $W_p$ = 0, IEE = 0 and the point $P_1(HR_{indv})$, the gradient of the regression line being found from the individual exercise heart rate found previously in the first step of the method at a defined bicycle ergometer exercise of 45 watt-hours and from the individual numerical measure ($MA_{indv}$) assigned to this exercise heart rate, **in that** the regression coefficients $P_0$ and $P_1$ describe at any physical exercise the connection between the insulin effect equivalents of physical exercise (IEE) generated on the basis of the model and any physical work ($W_p$) taking account of the individual exercise heart rate ($HR_{indv}$) and the intensity, the duration and the nature of the particular exercise, **in that** the influence of the individual exercise heart rate ($HR_{indv}$) is produced in accordance with the equation for exercise-specific numerical measure ($MA_{load}$) by the gradient, determined by the regression coefficient $P_1$, of the regression line between the physical work ($W_p$) and the exercise-specific numerical measure ($MA_{load}$) corresponding to this work depending functionally on the individual exercise heart rate ($HR_{indv}$) and thus being assigned in an individual-specific manner, and that the effect profile ($IEE(t)_{load}$) assigned to this exercise-specific numerical measure ($MA_{load}$) being determined in analogy to the procedure in the first step of the method in accordance with the relation

$$MA_{load} = F_1 * \int_{t_1}^{t_2} IEE(t)dt$$

using the differential equation system assigned to the glucose/insulin metabolism model, and finally the individual- specific exercise-dependent insulin effect equivalent

$$IEE_{load} = \int_{t_1}^{t_2} IEE(t)_{load}\,dt$$

of any physical exercise being found prospectively from the two variables.

**2.** Microcomputer arrangement for carrying out the method according to Claim 1, **characterized in that** its first distributed circuit, which serves to determine the individual-specific insulin effect equivalent on the basis of the exercise heart rate, consists in series of a first input module (1), of a heart rate module (2), of a regression module (3), of a first differential equation module (5) and of a first output module (6) and, arranged in parallel to this distributed circuit, is a second distributed circuit for determining the individual-specific insulin effect equivalent of any physical activities, which circuit is formed in series of a second input module (7) of a processing module (8), of a nomogram module (9), of a second differential equation module (10) and of a second output module (11), **in that** the output from the heart rate module (2) is fed to the second input of the nomogram module (9), and **in that** in each case one output from a model reference module (4) is connected to one input of the regression module, of the first and second differential equation module and of the nomogram module.

**Revendications**

**1.** Méthode pour la détermination des activités physiques individuelles spécifiques équivalentes à l'effet de l'insuline, **caractérisé en ce que**, pour les valeurs d'intensité, durée et type d'une charge physique, dans une première étape du procédé, une fréquence cardiaque de charge spécifique à l'individu ($FC_{indiv}$) est dérivée au moyen d'une constitution de valeurs moyennes des données de fréquence cardiaque de charge servant de données de saisie suivant le procédé pour une installation à micro-ordinateur et enregistrée dans l'installation à micro-ordinateur, qu'au moyen de cette fréquence cardiaque de charge spécifique à l'individu est déterminé, pour une charge physique définie, avec assistance informatique, l'équivalent insuline actif spécifique à l'individu ($EIA_{indv}$), consistant en une cote quantitative ($C_{indiv}$) et en un profil actif d'insuline ($EIA(t)_{indiv}$) associé à cette cote, en calculant, en utilisant la fréquence cardiaque de charge spécifique à l'individu, la cote quantitative ($C_{indiv}$), exprimée comme un multiple de l'effet d'une unité internationale d'insuline, de l'équivalent insuline actif spécifique à l'individu, ceci au moyen de l'équation de régression

$$C_{indiv} = K_0 - K_1 \, {}^*FC_{indiv},$$

le coefficient de régression $K_0$ signifiant la valeur numérique du point d'intersection entre les droites de régression et l'axe EIA et le coefficient de régression $K_1$ la valeur numérique de l'élévation de la droite de régression, et ceux-ci ayant été déterminés avant le début du procédé en condition clinique définie de test pour des diabétiques traités à l'insuline et les valeurs numériques des coefficients de régression étant enregistrés dans l'installation micro-informatique, que les coefficients de régression $K_0$ et $K_1$ décrivent la relation entre les équivalents actifs insuline (EIA) créés de manière assistée au moyen du modèle à métabolisme glucose/insuline avec une charge physique définie et la fréquence cardiaque de charge, et que ce profil actif associé à cette cote quantitative individuelle (EIA $(t)_{indiv}$) de l'étendue, de la durée et de l'évolution dans le temps des effets analogues à l'insuline est déterminé à partir de la relation définie par le modèle à métabolisme glucose/insuline entre la cote quantitative et la surface dans l'évolution des effets équivalents insuline en fonction du temps de la charge physique (EIA(t)) suivant la relation

$$C_{indv} = F_1{}^* \int_{t_1}^{t_2} EIA(t)\,dt$$

le facteur de proportionnalité $F_1$ servant à décrire la caractéristique de dose/effet pour la détermination du multiple de l'effet équivalent insuline de la charge physique définie en relation avec une unité d'insuline administrée, que l'évolution de l'effet équivalent insuline en fonction du temps de la charge physique (EIA(t)) est simulée de manière assistée par le modèle par inversion entrée/sortie au moyen d'une intégration itérative du système d'équation différentielle associé au modèle à métabolisme glucose/insuline

$$x' = u + CHO(t),$$

$$u' = - (b_1 + b_2)u — b_3(y + e) + b_1(b_0 — CHO(t)),$$

$$y' = - ky + INS(t),$$

$$e' = - k \, e + EIA(t),$$

en définissant comme grandeurs d'état x = évolution de la concentration de glucose dans le sang, u = bilan de glucose endogène, y = concentration d'insuline dans le sang et e = l'effet équivalent insuline de la charge physique ainsi qu'en tant que grandeurs d'entrée en fonction du temps du modèle
CHO(t) = évolution dans le temps de la résorption de nourriture,
INS(t) = évolution dans le temps de l'effet réducteur de sucre dans le sang de l'insuline,
$b_i$ et k = paramètres pour la production endogène de glucose ($b_0$),
$b_1$ et $b_2$ = facteur de renforcement et constante de temps de la conversion de glucose dépendant de l'insuline,
$b_3$ = exploitation du glucose stimulant l'insuline et
k = constante de temps du catabolisme de l'insuline,
et déterminée par comparaison de l'évolution de l'effet équivalent insuline en fonction du temps de la charge physique (EIA(t)) avec le profil, connu pour une application sous-cutanée définie d'insuline, de l'effet réducteur de sucre dans le sang de l'insuline (INS(t)), et que l'équivalent insuline actif spécifique à l'individu ($EIA_{indiv}$) est calculé pour la charge physique définie à partir de la relation

$$EIA_{indv} = \int_{t_1}^{t_2} EIA(t)_{indv} dt$$

la cote quantitative individuelle correspondant à l'intégrale de la surface sous la courbe de l'évolution individuelle en fonction du temps de l'effet de l'insuline, que, dans une deuxième étape du procédé, l'équivalent insuline actif de n'importe quelle charge physique ($EIA_{charge}$), consistant en une cote quantitative ($C_{charge}$) et en le profil actif d'insuline associé à cette cote ($EIA(t)_{charge}$) est déterminé en saisissant au début dans l'installation micro-informatique les valeurs d'intensité (I) et de durée (D) et le type (A) d'une charge physique, qu'à partir des grandeurs d'entrée d'abord la puissance de travail ($AL_k$) à fournir pour l'activité physique respective, exprimée en watts-heure, est calculée suivant la relation

$$AL_k = A_i * (I * D),$$

par multiplication de l'intensité (I) et de la durée (D) de la charge physique, le type de charge physique étant considéré sous forme du facteur de multiplication ($A_i$) sans dimensions qui est enregistré sous forme de tableau dans l'installation micro-informatique, que, pour la puissance de travail ($AL_k$) ainsi calculée d'une charge physique, au moyen d'un nomogramme, la cote correspondant à cette puissance de travail ($C_{charge}$) est déterminée par régression suivant l'équation

$$C_{charge} = P_0 + P_1(FC_{indv}) * AL_k,$$

les valeurs numériques pour les coefficients de régression $P_0$ et $P_1$, qui sont créées et enregistrées dans l'installation micro-informatique, résultant de l'élévation de la droite de régression du propositus respectif entre le point $P_0$ pour $AL_k = 0$, EIA = 0 et le point $P_1(FC_{indv})$, l'élévation de la droite de régression étant déterminée à partir de la fréquence cardiaque de charge individuelle déterminée préalablement dans une première étape du procédé avec une charge d'ergomètre cycliste définie de 45 watts-heure et de la cote individuelle ($C_{indiv}$) associée à cette fréquence cardiaque de charge, que les coefficients de régression $P_0$ et $P_1$ décrivent avec n'importe quelle charge physique la relation entre les équivalents insuline actifs de charge physique (EIA) élaborés avec assistance par modèle et n'importe quelle puissance de travail physique ($AL_k$), en tenant compte de la fréquence cardiaque de charge individuelle ($FC_{indiv}$) et de l'intensité, de la durée et du type de la charge respective, que l'influence de la fréquence cardiaque de charge individuelle ($FC_{indiv}$) est réalisée en fonction de l'équation pour cette spécifique à la charge ($C_{charge}$) par le fait que l'élévation définie par les coefficients de régression $P_1$ des droites de régression entre la puissance de travail physique ($AL_k$) et la cote correspondant à cette puissance de travail ($C_{charge}$) dépend fonctionnellement de la fréquence cardiaque de charge individuelle ($FC_{indiv}$) et est ainsi associée spécifiquement à l'individu et que le profil d'effet ($EIA(t)_{charge}$) associé à cette cote spécifique à la charge ($C_{charge}$) est défini en utilisant le système d'équation différentielle associé au modèle à métabolisme glucose/insuline de manière analogue à la procédure de la première étape du procédé suivant l'équation

$$C_{charge} = F_1 * \int_{t_1}^{t_2} EIA(t)dt$$

et enfin, à partir des deux grandeurs, l'équivalent insuline actif dépendant de la charge spécifique à l'individu

$$EIA_{charge} = \int_{t_1}^{t_2} EIA(t)_{charge}dt$$

de n'importe quelle charge physique est déterminé de manière prospective.

2. Installation micro-informatique pour la réalisation du procédé selon la revendication 1, **caractérisée en ce que** son premier circuit en chaîne, qui sert à la définition de l'équivalent insuline actif spécifique à l'individu sur la base de la fréquence cardiaque de charge consiste en série en un premier module d'entrée (1), un module de fréquence cardiaque (2), un module de régression (3), un premier module à équation différentielle (5) et un premier module de sortie (6) et, parallèlement à ce circuit en chaîne, est disposé un deuxième circuit en chaîne servant à la définition de l'équivalent insuline actif spécifique à l'individu de n'importe quelle activité physique, qui est composé en série d'un deuxième module d'entrée (7), d'un module de préparation (8), d'un module de nomogramme (9), d'un deuxième module à équation différentielle (10) et d'un deuxième module de sortie (11), que la sortie du module de fréquence cardiaque (2) est guidée vers la deuxième entrée du module de nomogramme (9), et que chaque sortie d'un module de référence de modèle (4) est branchée à une entrée du module de régression, du premier et du deuxième module d'équation, différentielle et du module de nomogramme.

Fig. 1

Fig. 2

EP 0 824 240 B1

Fig. 3

EP 0 824 240 B1

Fig. 4

Fig. 5

Fig. 6